Europäisches Patentamt

European Patent Office

Office européen des brevets

(11) Numéro de publication: **0 206 920**
A1

## (12) DEMANDE DE BREVET EUROPEEN

(21) Numéro de dépôt: 86401302.4

(22) Date de dépôt: 16.06.86

(51) Int. Cl.⁴: **C 12 N 15/00,** C 12 P 21/02, A 61 K 45/02, A 61 K 35/76, C 12 N 5/00, C 12 N 7/00

(30) Priorité: 18.06.85 FR 8509225

(43) Date de publication de la demande: 30.12.86
Bulletin 86/52

(84) Etats contractants désignés: **AT BE CH DE FR GB IT LI LU NL SE**

(71) Demandeur: **TRANSGENE S.A., 16, rue Henri Regnault, F-92400 Couirbevoie (FR)**

(72) Inventeur: **Faure, Thérèse, 67 bis, route des Romains, F-67200 Koenigshoffen (FR)**
Inventeur: **Sondermeyer, Paul, 24, rue des Accacias, F-67400 Ostwald (FR)**
Inventeur: **Altenburger, Werner, Esterli Weg 135, CH-1425 Riehen-Bâle (CH)**
Inventeur: **Lecocq, Jean-Pierre, 6, rue du Champ du Feu, F-67116 Reichstett (FR)**

(74) Mandataire: **Warcoin, Jacques et al, Cabinet Régimbeau 26, avenue Kléber, F-75116 Paris (FR)**

(54) Vecteur d'expression de l'interféron- gamma dans les cellules de mammifère, procédé pour sa mise en oeuvre, produit obtenu et composition pharmaceutique contenant l'interféron- gamma.

(57) L'invention concerne un virus de la vaccine, caractérisé en ce qu'il comporte tout ou partie d'une séquence d'ADN codant pour IFN-γ.

L'utilisation de ce virus pour infecter des cellules de mammifères conduit à des cellules productrices d'IFN-γ qui peut être utilisé dans des compositions thérapeutiques.

EP 0 206 920 A1

**"Vecteur d'expression de l'interféron-γ dans les cellules de mammifère, procédé pour sa mise en oeuvre, produit obtenu et composition pharmaceutique contenant l'interféron-γ"**

Les interférons humains ont été décrits par Isaacs et Lindenmann (1957) comme un groupe de protéines capables d'induire un état de résistance aux virus dans les cellules cibles.

Depuis, de nombreuses activités biologiques importantes ont été décrites (voir Stewart, 1979), notamment la modulation immunitaire et l'inhibition de la prolifération cellulaire, de même que des effets directs sur la croissance tumorale chez les animaux.

C'est pourquoi on espère que dans un futur proche les interférons joueront un rôle important dans le traitement clinique des infections virales et des tumeurs.

Parmi les trois classes d'interférons humains, leucocytaire (IFN-α), fibroblastique (IFN-β) et immun (IFN-γ), l'IFN-γ a démontré qu'il était le plus actif dans ses propriétés immunorégulatrices (Sonnenfeld et coll. 1977), de régulation cellulaire (Blalock et coll., 1980) et d'inhibition des tumeurs (Salvin et coll., 1975 ; Glasgow et coll., 1978).

L'IFN-γ naturel est induit dans des cultures de lymphocytes de sang périphérique par stimulation mitogénique. C'est seulement récemment que Rinderknecht et coll. (1984) ont décrit la séquence correcte de la protéine mature et montré son type et son degré de glycosylation.

La séquence protéique déduite des expériences de clonage de cADN a conduit à diverses ambiguités concernant les extrémités N- et C-terminales de la molécule biologiquement active mature. Par exemple, la séquence de la protéine choisie pour être exprimée dans Escherichia coli et la levure et basée sur les informations sur le cADN publiées par Gray et coll. (1982) ne correspond pas exactement à la séquence naturelle, bien que ces modifications n'affectent pas l'activité biologique de la molécule.

La présente invention concerne un système reposant sur un virus recombinant de la vaccine (VV) ayant incorporé le gène codant pour la protéine IFN-γ et qui permet la synthèse efficace d'une protéine qui est libérée dans le milieu de culture. Cette synthèse peut être obtenue dans différents types de cellules eucaryotes. En outre, ce virus peut être propagé dans les animaux vivants, ce qui permet l'étude in vivo de l'effet immunorégulateur d'un taux élevé d'IFN-γ endogène.

Plusieurs groupes ont récemment mis en évidence l'utilisation de recombinants vivants du virus de la vaccine pour exprimer un antigène de l'Influenza, de l'Hépatite B et    la glycoprotéine de la rage pour immuniser contre ces maladies ou infections (Smith et coll. 1983; Panicali et coll., 1983; Kieny et coll. 1984).

L'expression d'une séquence codante pour une protéine exogène dans le virus de la vaccine (VV) implique nécessairement deux étapes :

1) La séquence codante doit être alignée avec un promoteur de VV et être insérée dans un segment non essentiel de l'ADN de VV, cloné dans un plasmide bactérien approprié.

2) Les séquences d'ADN de VV situées de part et d'autre doivent permettre des recombinaisons homologues in vivo entre le plasmide et le génome viral. Une double recombinaison réciproque conduit à un transfert de l'insert d'ADN du plasmide dans le génome viral par lequel il sera propagé et exprimé (Panicali et Paoletti, 1982; Mackett et coll., 1982; Smith et coll., 1983; Panicali et coll., 1983).

La présente invention concerne un poxvirus, en particulier un virus de la vaccine, caractérisé en ce qu'il comporte la séquence d'ADN codant pour la protéine IFN-γ humaine. Ce virus comportera de préférence l'ensemble des éléments assurant l'expression d'un IFN-γ mature complet qui sera sécrété par les cellules dans le milieu.

En particulier, il comportera un promoteur d'un gène de poxvirus, en particulier de la vaccine, situé en amont de la séquence codant pour la protéine IFN-γ humaine, tel que le promoteur du gène de 7,5 k, noté P 7,5 k, qui assurera l'expression de la séquence d'ADN codant pour IFN-γ nommée ci-après d'ADN (IFN-γ).

Cet ensemble promoteur/séquence d'ADN (IFN-γ) sera inséré dans une séquence non essentielle de l'ADN de la vaccine telle qu'un gène du virus de la vaccine, par exemple le gène TK, ce qui fournit une possibilité de sélection comme cela sera expliqué ci-après.

Bien entendu, dans le cadre de la présente invention, lorsque l'on parle de "virus de la vaccine" il peut s'agir du virus entier ou d'un virus dont certaines parties non essentielles ont été délétées.

L'invention concerne également des cellules de mammifère infectées par un virus tel que défini précédemment.

Parmi les cellules particulièrement utilisables, il faut citer notamment les cellules Vero, bien que d'autres cellules puissent être utilisées, comme cela sera décrit dans les exemples.

L'invention concerne également un procédé de préparation d'IFN-γ humain obtenu par culture des cellules décrites précédemment dans un milieu de culture approprié.

4

0206920

L'invention concerne, enfin, la protéine IFN-γ humaine obtenue par la mise en oeuvre dudit procédé.

L'invention concerne, en particulier les compositions thérapeutiques contenant à titre de principe actif l'IFN-γ obtenu par la mise en oeuvre du procédé selon l'invention, ainsi que les compositions thérapeutiques contenant à titre de principe actif un poxvirus, en particulier un virus de la vaccine, modifié comme cela a été décrit précédemment. Dans ce dernier cas, le virus de la vaccine utilisé comme agent thérapeutique de traitement du corps humain ou d'un animal doit permettre la formation de l'IFN-γ in vivo.

La préparation du virus recombinant selon l'invention comporte, notamment, les étapes suivantes :

1) Une restructuration d'une extrémité du cADN (IFN-γ) pour obtenir M13pTGO5 ;

2) La synthèse d'un mini-plasmide pTG1H à partir de pBR322 ;

3) L'insertion dans ce mini-plasmide du fragment Hin-J portant le gène TK de VV ;

4) L'insertion dans le gène TK du promoteur de la protéine 7,5 k ;

5) L'insertion d'un polylinker en aval du promoteur P 7,5 k ;

6) L'insertion entre deux sites de restriction du polylinker de la séquence de cADN complète codant pour la protéine IFN-γ ;

7) Le clonage des éléments essentiels de ce dernier plasmide dans le virus de la vaccine.

Les exemples suivants illustrent les propriétés des différents composants obtenus.

Les différents matériels mis en oeuvre sont identifiés dans les exemples.

Sauf indication contraire, les enzymes sont utilisées dans les conditions préconisées par le fabricant et les techniques mises en oeuvre sont connues de l'homme de métier.

Les séquences d'acides aminés et les séquences de nucléotides représentées sur les figures ne sont pas reprises dans le corps de la description pour ne pas l'alourdir mais elles en constituent une partie intégrante.

Sur les figures ci-annexées,

la figure 1 représente la séquence du cADN de pTG11 codant pour la protéine IFN-γ,

la figure 2 schématise la mutation du cADN précédent pour introduire un site BglII,

la figure 3 schématise la structure de pML2,

la figure 4 schématise la préparation de pTG186-POLY,

la figure 5 schématise la préparation de pVVTG41,

la figure 6 schématise le clonage dans le virus de la vaccine,

la figure 7 représente la fluorographie des fragments d'ADN de virus recombinant TK$^-$ après hybridation avec une sonde cADN(IFN-γ) marquée,

la figure 8 représente la courbe de la cinétique de synthèse et de libération dans le milieu de l'IFN-γ par des cellules infectées par le virus recombinant vaccine IFN-γ à $3.10^{-2}$ MOI.

1 - Construction des vecteurs

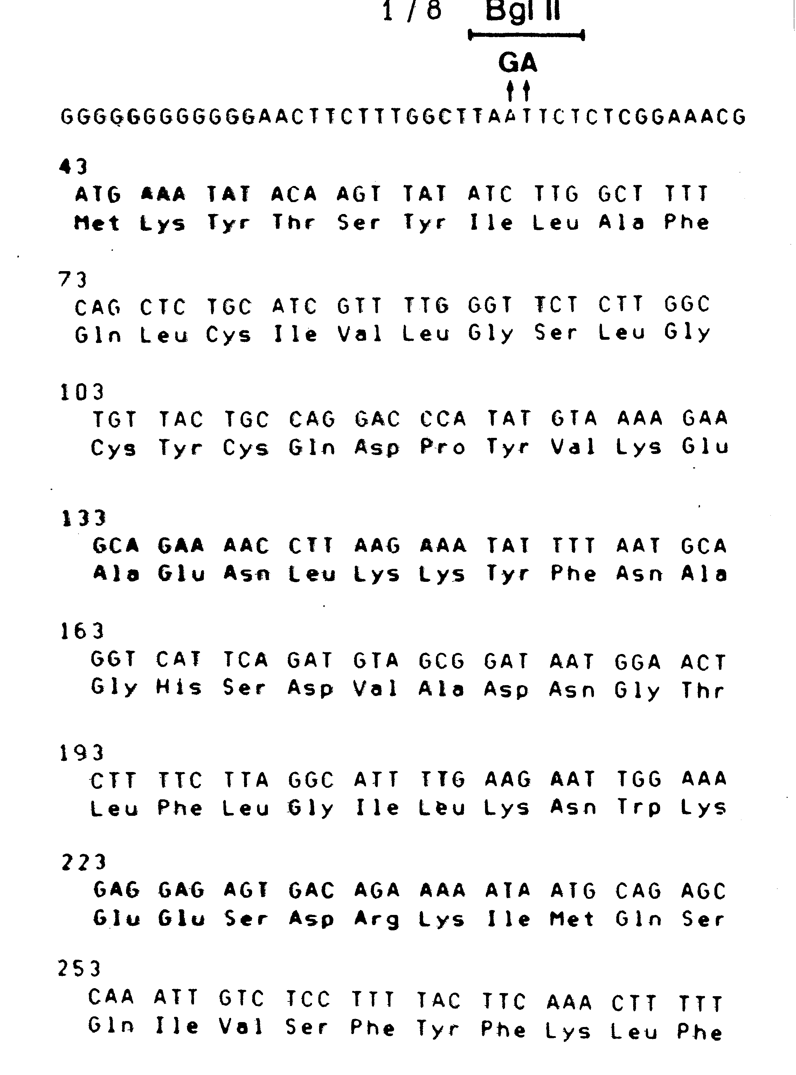

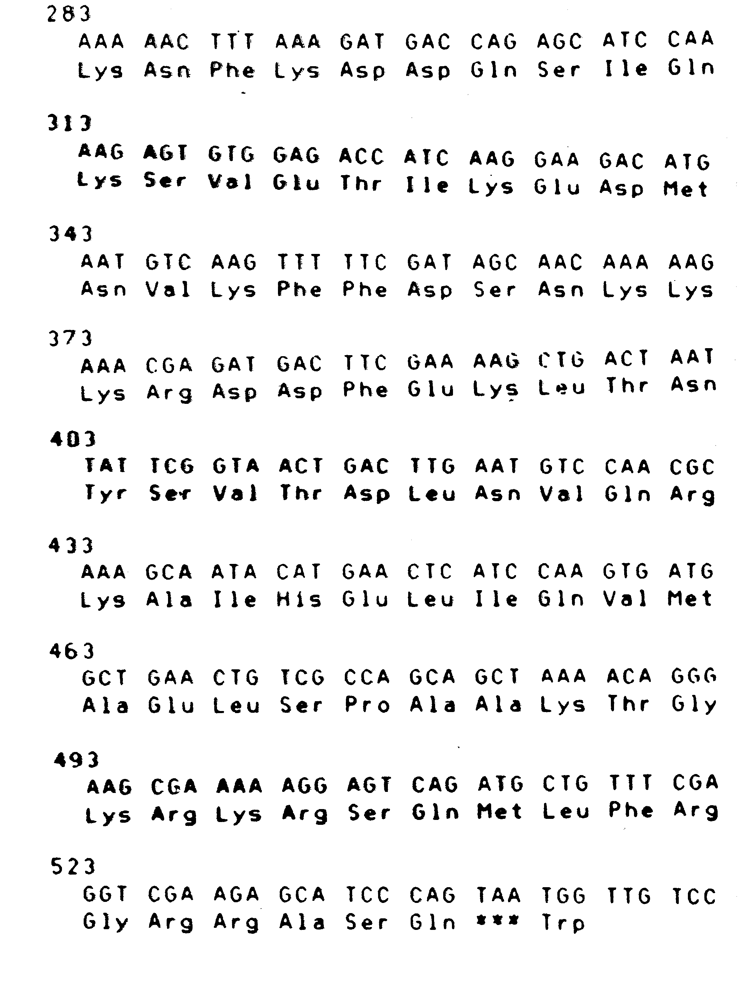

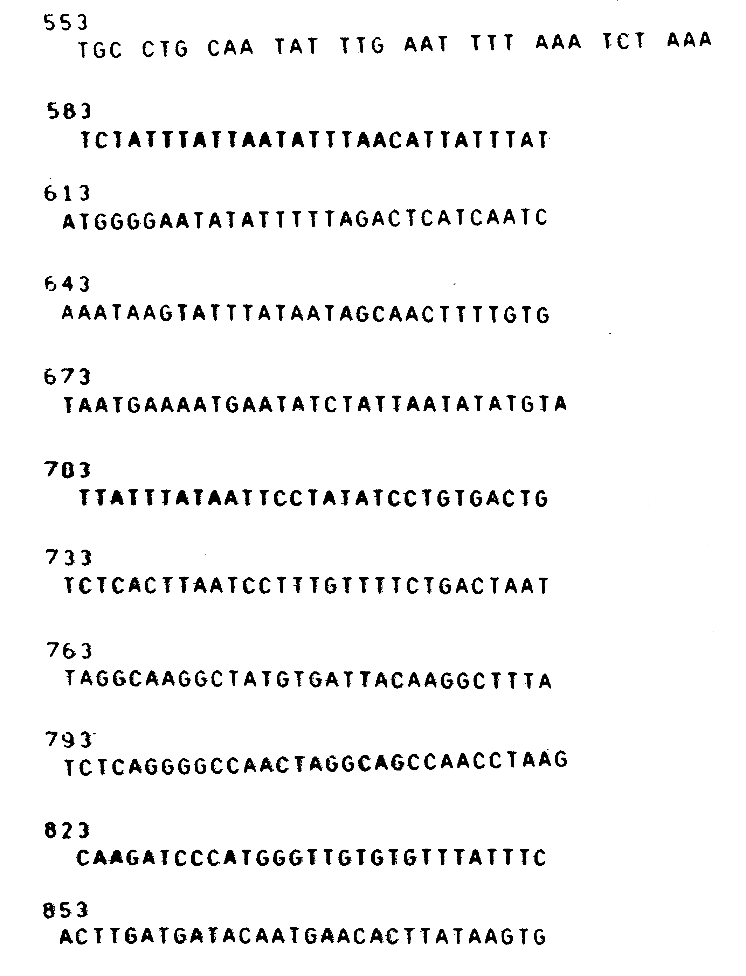

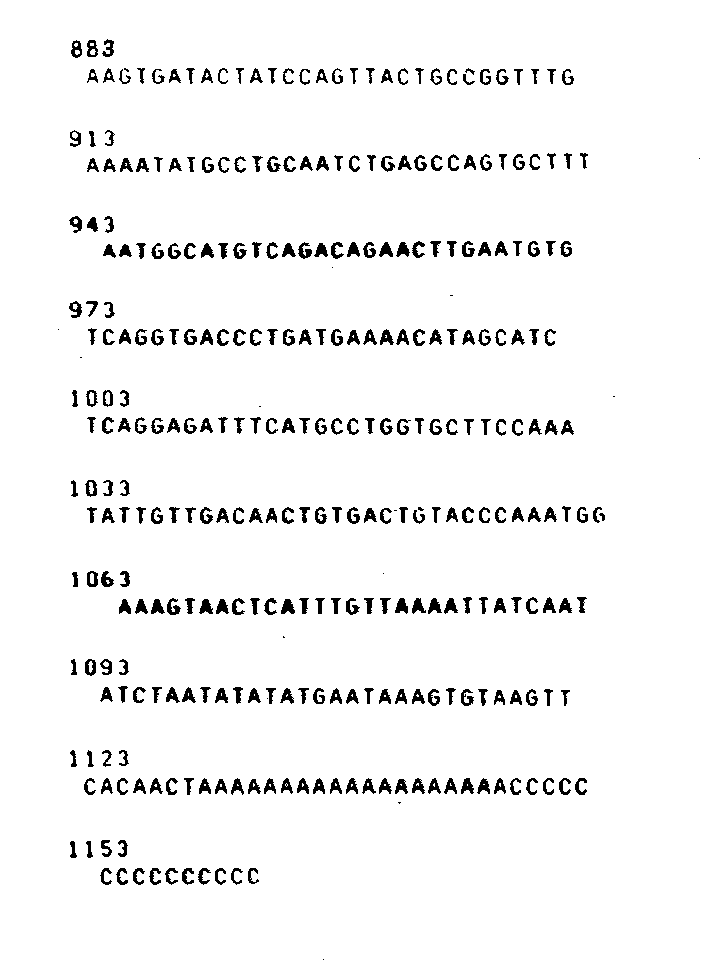

La séquence de cADN du clone pTG11, isolé à l'origine à partir d'une banque provenant d'ARN de lymphocytes stimulés par mitogénèse, est donnée à la figure 1.

Cette séquence diffère de la première séquence publiée par Gray et coll. (1982) à la position 521. Le triplet CAA codant pour Gln est remplacé dans pTG11 par le triplet CGA qui code pour Arg. En effet, de nombreux groupes ont confirmé cette mutation et, en outre, la séquence génomique montre une guanidine à la place de l'adénine qui avait été décrite.

La séquence codant pour IFN-γ dans pTG11 est précédée par 30 bases de la région non codante de l'extrémité 5'.

Afin d'écarter la présence de l'élongation poly-dG/dC entre le promoteur P7,5 k et le départ de la séquence de la protéine dans la futureconstruction, on introduit un site de restriction BglII en amont du codon de départ, ce qui facilitera également le transfert du cADN dans le vecteur VV.

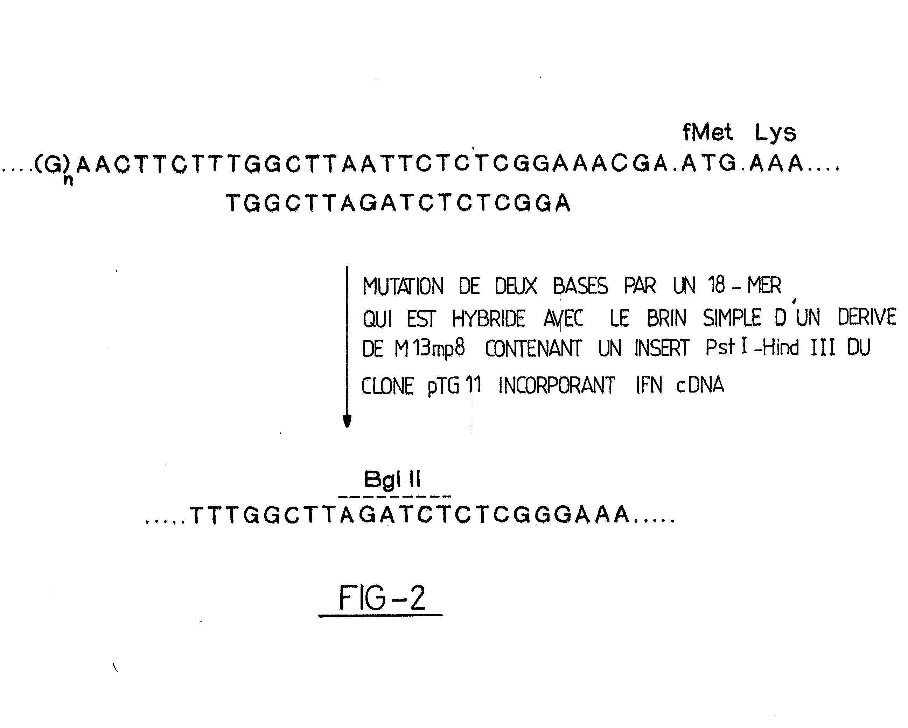

La mutation spécifique du site est effectuée selon le schéma représenté à la figure 2.

On insère dans l'ADN bicaténaire du phage M13mp8 un fragment de restriction PstI-HindII de 1100 bp provenant du cADN de IFN-γ contenu dans pTG11. L'ADN mono-caténaire dérivé de M13mp8 est hybridé avec un oligomère synthétique de 18 nucléotides ayant la structure représentée sur la figure 2, qui remplace une séquence AT par une séquence

On obtient ainsi un dérivé du phage M13 nommé M13pTGO5 qui par digestion avec BglII et EcoRI conduit à un fragment de 950 bp ne contenant plus d'extrémité dG/dC et codant pour la protéine IFN-γ complète.

## 2) Construction des plasmides hybrides

Les tailles combinées des différents éléments nécessaires pour le transfert de la séquence codant pour l'IFN-γ humain dans le génome de VV et son expression subséquente sont de l'ordre de plusieurs kb. Il a donc été jugé nécessaire de minimiser la taille du plasmide de réplication dans E. coli utilisé pour le travail de construction, de façon à faciliter les manipulations nécessaires.

Le fragment HindIII (Hin-J) du génome de VV contient le gène complet de la thymidine kinase (TK) qui a déjà été utilisé précédemment pour permettre l'échange et la recombinaison d'ADN étranger dans le génome de VV (Mackett et coll., 1982). Il est important de noter que le transfert d'un insert dans le gène TK de VV crée un virus TK déficient qui peut être reconnu. Il a tout d'abord été nécessaire de produire un plasmide de petite taille portant un site unique HindIII utilisable pour l'intégration du fragment Hin-J de l'ADN de VV. En outre, il était nécessaire d'éliminer les séquences de restriction non nécessaires du plasmide de façon à permettre les manipulations suivantes.

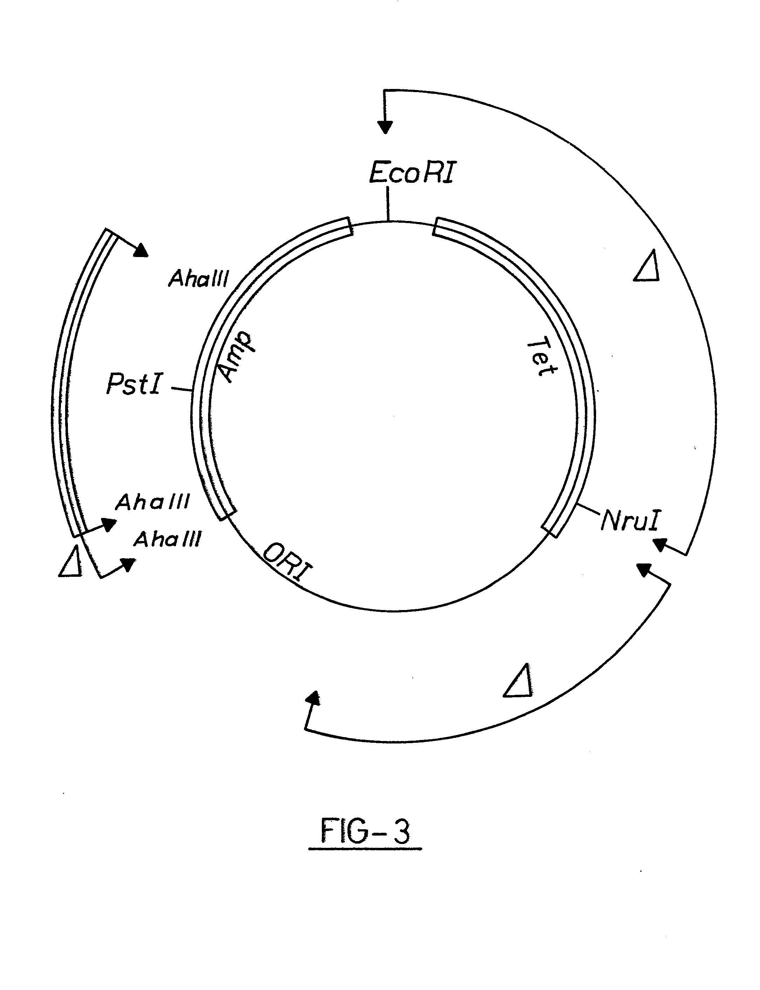

La construction a été amorcée à partir du plasmide pML2 (Lusky et Botchan, 1981) qui est un vecteur dérivé du plasmide pBR322 par délétion spontanée du segment entre les nucléotides 1089 et 2491 (figure 3). D'abord la séquence de PstI a été éliminée par insertion du fragment AhaIII-AhaIII de pUC8 (Vieira et Messing, 1982) entre deux sites AhaIII de pML2 en éliminant 19 paires de bases. On a utilisé la méthode de "linker-tailing" (Lathe et coll., 1984) pour insérer dans ce plasmide un linker HindIII entre les sites NruI et EcoRI préalablement traités par la S1. Ceci conduit à un plasmide de 2049 paires de bases ne comportant plus de site BamHI et portant le gène β-lactamase fonctionnel (conférant la résistance à l'ampicilline) et comportant, en outre, une origine de réplication active dans E. coli et un site de restriction unique HindIII.

Cette construction a été appelée pTG1H.

3)

Le fragment Hin-J de l'ADN de VV portant le gène TK a préalablement été cloné dans un vecteur provenant de pBR327 (Drillien et Spehner, 1983). Ce fragment de 4,6 kb a été recloné dans le site HindIII de pTG1H. Un clone a été sélectionné dans lequel le gène TK est situé

8

0206920

distalement par rapport au gène codant pour la résistance à l'ampicilline.

Cette construction pTG1H a été utilisée comme porteur dans l'expérience suivante.

L'étape suivante a été d'isoler un promoteur de VV utilisable pour commander l'expression de la séquence codant pour IFN-γ humain. Le promoteur d'un gène précoce codant pour une protéine de 7500 daltons (7,5 k) a déjà été utilisé avec succès dans un but identique (Smith et coll., 1983) et on a donc procédé à l'isolement de ce segment.

Le gène 7,5 k est situé sur l'un des plus petits fragments SalI (fragment Sal-S) du génome de VV type WR (Venkatasan et coll., 1981). Comme les petits fragments sont clonés de façon préférentielle, une grande proportion des clones obtenus par clonage direct de l'ADN de VV type WR coupé par SalI dans le plasmide pBR322 porte le fragment Sal-S. Ce fragment est inséré dans le bactériophage vecteur M13mp701 (Kieny et coll., 1983) par digestion SalI et religation, ce qui donne le phage M13TGSal-S.

Dans ce clone, un site ScaI se trouve immédiatement à proximité de l'ATG d'initiation du gène 7,5 K. En aval du gène 7,5 K se trouvent situés des sites uniques BamHI et EcoRI provenant du vecteur. Les sites BamHI et ScaI sont fusionnés par l'intermédiaire d'un linker BglII 5'-CAGATCTG-3' après avoir complété les extrémités générées par digestion BamHI avec le fragment Klenow de la poly-mérase. Ce procédé élimine le site ScaI mais reconstitue le site BamHI et déplace le site unique EcoRI en aval. En même temps, le site SalI (AccI) en aval est éliminé, le site en amont devient donc unique.

Cette construction est appelée M13TG7,5K 4)

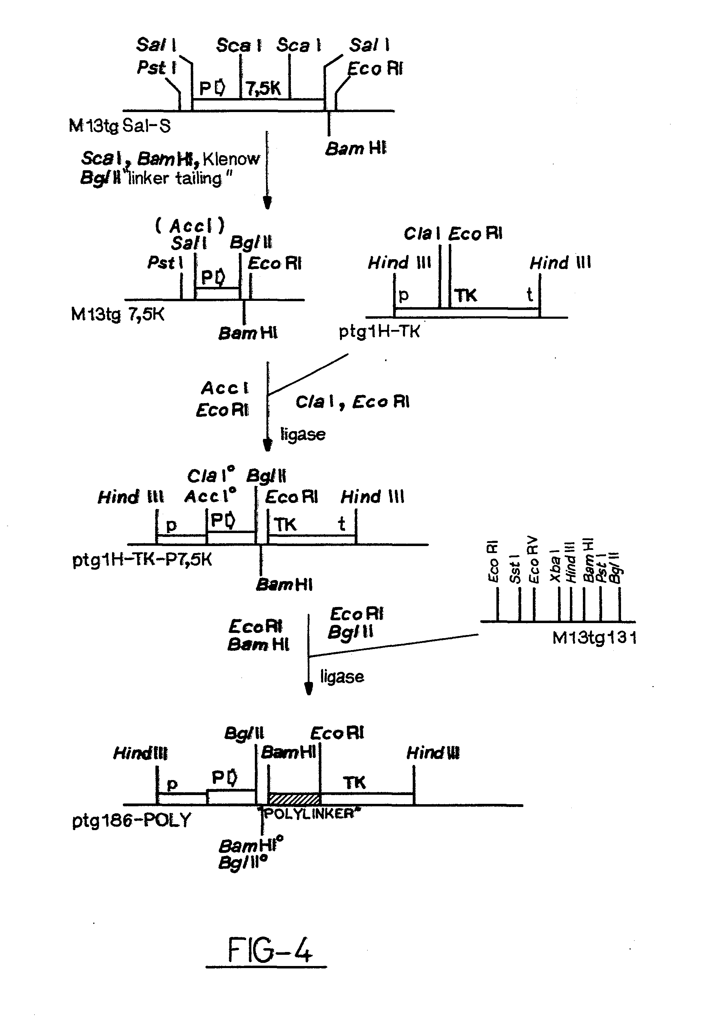

A l'intérieur du fragment Hin-J de l'ADN de VV se trouvent situés des sites ClaI et EcoRI qui sont séparés par environ 30 paires de bases (Weir et Moss, 1983). Le fragment promoteur de 7,5 K présent dans M13TG7,5K est excisé par AccI et EcoRI et cloné entre les sites ClaI et EcoRI de pTG1H-TK pour générer pTG1H-TK-P7,5K dont la synthèse est schématisée dans la figure 4.

Cette construction conduit au transfert des sites BamHI et EcoRI uniques du vecteur M13 immédiatement en aval de la séquence du promoteur 7,5K. Ces sites uniques BamHI et EcoRI sont utilisés dans la construction suivante.

5) Le segment polylinker du bactériophage M13TG131 (Kieny et coll., 1984) est excisé par EcoRI et BglII et inséré entre les sites EcoRI et BamHI du plasmide pTG1H-TK-P7,5K générant pTG186-POLY. Dans cette construction, 10 sites de restriction sont disponibles pour le clonage d'un gène étranger sous le contrôle de P7,5K.

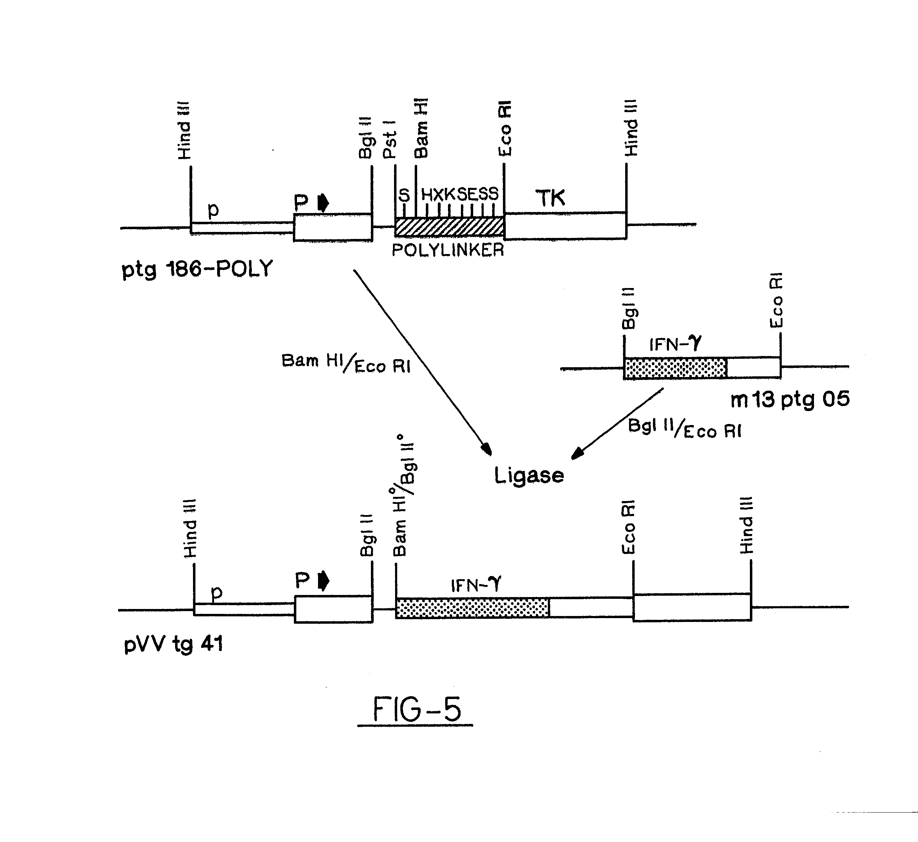

6) pTG186-POLY est mis en digestion avec BamHI et EcoRI et ligué avec le fragment BglII-EcoRI venant du M13pTG05 et contenant la séquence de cADN IFN-γ (figure 5). Après transformation de E. coli, les plasmides recombinants sont vérifiés par digestion enzymatique de minipréparations d'ADN plasmidique. Un candidat correct a été retenu et appelé pVVTG41.

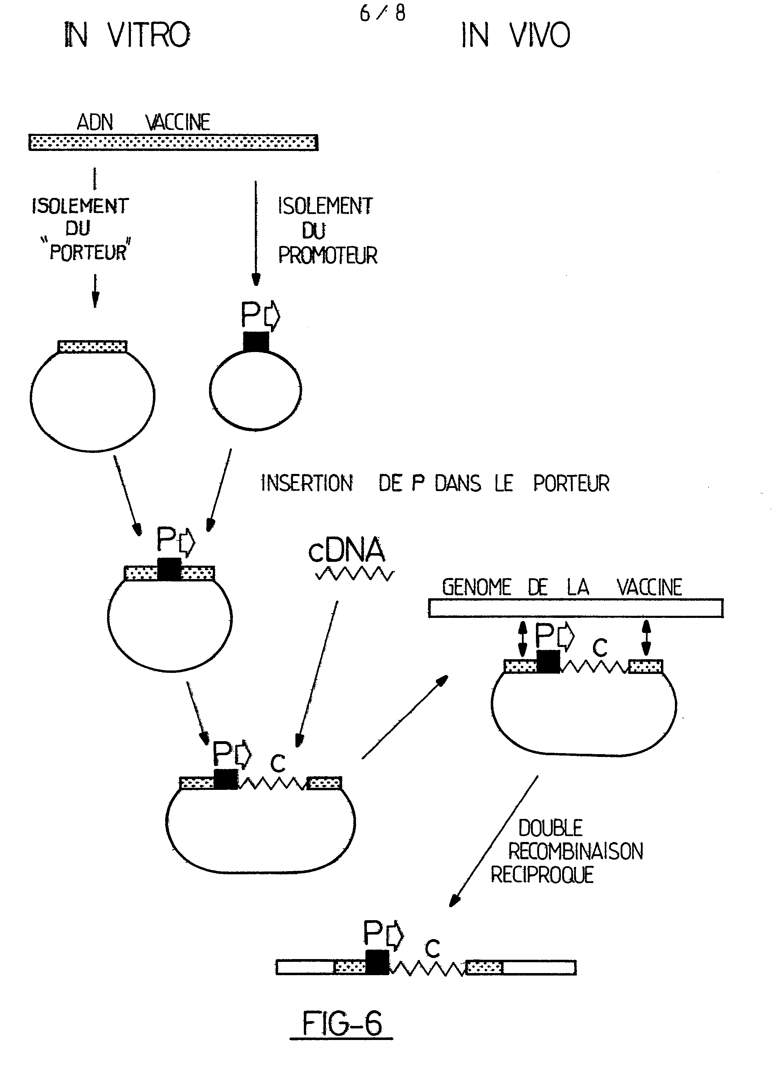

7) <u>Clonage dans le virus de la vaccine (figure 6)</u>

La stratégie décrite par Smith et coll. (1983) repose sur l'échange in vivo entre un plasmide portant un insert dans le gène VV TK et le génome viral de type sauvage de façon à inactiver le gène TK porté par le virus. Les virus TK⁻ peuvent être sélectionnés par étalement sur un lignée cellulaire (TK-négative) en présence de 5-bromodéoxyuridine (5BUDR) (Mackett et coll., 1982). La thymidine kinase phosphoryle le 5BUDR en 5'-monophosphate, qui est ensuite converti en triphosphate. Ce composé est un analogue de dTTP et son incorporation dans l'ADN bloque le développement correct du virus. Un virus TK⁻ peut néanmoins répliquer son ADN normalement et il conduit à des plaques virales visibles dans une lignée cellulaire également TK⁻.

Le virus de la vaccine se propage dans le cytoplasme des cellules infectées plutôt que dans leur noyau. C'est pourquoi il n'est pas possible de tirer avantage de la machinerie de réplication et de transcription de l'ADN de l'hôte et il est nécessaire que le virion possède les composants pour l'expression de son génome. L'ADN de VV purifié est non-infectieux.

Afin de générer les recombinants, il est nécessaire d'effectuer simultanément l'infection cellulaire avec du virion VV et une transfection avec le segment d'ADN cloné qui présente de l'intérêt. Toutefois, la génération des recombinants est limitée à la petite proportion des cellules qui sont compétentes pour la transfection par l'ADN. C'est pour cette raison qu'il a été nécessaire de mettre en oeuvre une stratégie de "congruence" indirecte pour réduire le bruit de fond des virus parentaux non-recombinants. Ceci a été effectué en utilisant comme virus infectieux vivant un mutant thermosensible (ts) de la vaccine qui n'est pas capable de se propager à une température non permissive de 39,5°C (Drillien et Spehner, 1983). Lorsque les cellules sont infectées avec un mutant ts dans des conditions non permissives et transfectées avec l'ADN d'un virus de type sauvage, la multiplication virale interviendra seulement dans les cellules qui sont compétentes pour la transfection et dans lesquelles une recombinaison entre l'ADN viral sauvage et le génome du virus ts aura eu lieu ; aucun virus ne se multipliera dans les autres cellules, en dépit du fait qu'elles ont été infectées. Si un plasmide recombinant contenant un fragment de l'ADN de vaccine tel que pVVTG41 est inclus dans le mélange de transfection,à la concentration appropriée,avec l'ADN du type sauvage, il est également possible d'obtenir qu'il participe à la recombinaison homologue avec l'ADN de la vaccine dans les cellules compétentes.

Des monocouches de cellules primaires de fibroblastes d'embryons de poulets (CEF) sont infectées à 33°C avec VV-Copenhague ts7 (0,1 pfu/cellule) et transfectées avec un coprécipité au phosphate de calcium de l'ADN du virus de type sauvage VV-Copenhague (50 ng/$10^6$ cellules) et le plasmide recombinant (25-30 ng/$10^6$ cellules).

Après incubation pendant 2 heures à une température qui ne permet pas le développement du virus ts (39,5°C), les cellules sont incubées de nouveau pendant 48 heures à 39,5°C. Des dilutions de virus ts$^+$ sont utilisées pour réinfecter une monocouche de cellules de souris L-TK$^-$ à 37°C qui sont ensuite incubées en présence de 5BUDR (100 µg/ml). Différentes plaques de virus TK$^-$ sont obtenues à partir de ces cellules qui ont reçu le plasmide recombinant, tandis que les cultures contrôles sans plasmide ne montrent pas de plaques visibles. Les virus TK$^-$ sont ensuite sous-clonés par une deuxième sélection en présence de 5BUDR.

Une double recombinaison réciproque correcte entre le plasmide hybride IFN-γ/vaccine et le génome de VV aboutit à l'échange du gène TK portant l'insert avec le gène TK du virus, les recombinants devenant ainsi TK⁻.

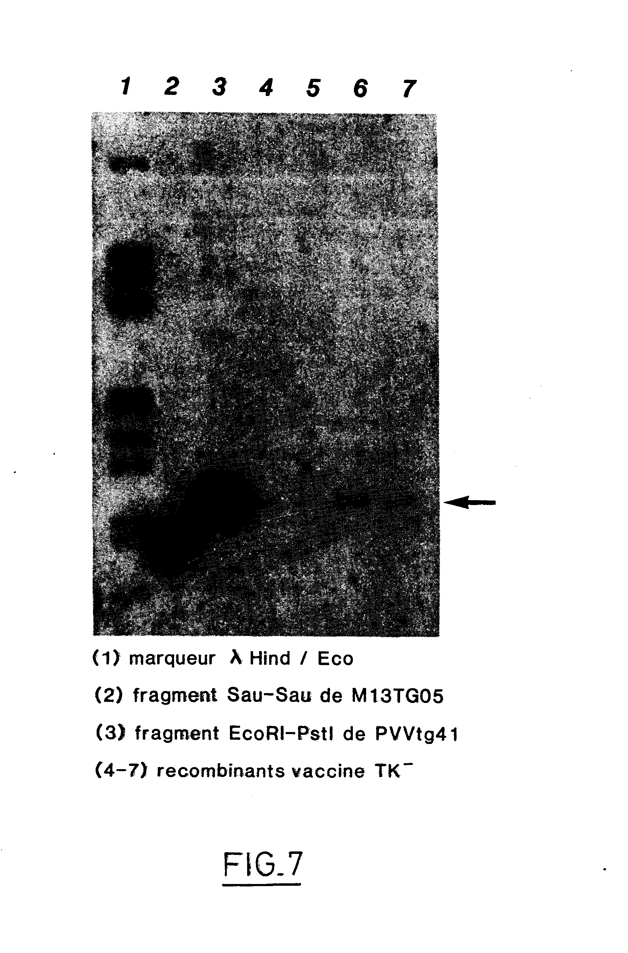

Les ADN purifiés à partir des différents virus recombinants TK⁻ sont digérés simultanément par PstI et EcoRI et soumis à une électrophorèse sur gel d'agarose. Les fragments d'ADN sont transférés sur un filtre de nitrocellulose selon la technique décrite par Southern (1975). Le filtre est ensuite hybridé avec une sonde cADN (IFN-γ), nick-translaté au $^{32}$P. Après lavage du filtre, ce dernier est fluorographié et une bande de 1,0 kb est visible sur l'autoradiographie quand le virus vaccine a incorporé le gène IFN-γ (figure 7).

## Expression de l'interféron-γ humain après infection des cellules de mammifères avec un virus de la vaccine recombinant

Un virus TK⁻ recombinant pVVTG41 ayant intégré le gène IFN-γ est utilisé pour infecter différentes espèces de cellules en culture.

Les lignées humaines (Chang, HeLa),murine (LM), de hamster (BHK), canine (MDCK), de singe (Vero) ou des cellules primaires de poulet sont testées.

Toutes ces lignées peuvent produire des quantités d'interféron-γ humain voisines.

Un protocole type ainsi que les résultats obtenus sont décrits ici pour la lignée Vero.

## Infection des cellules Vero par la vaccine

Des monocouches de cellules Vero (80 % à 100 % confluentes) sont infectées par le virus à différentes multiplicités d'infection (MOI entre 10 et $10^{-5}$ pfu/cell.)

en milieu dépourvu de sérum. Après adsorption pendant 1 heure à température ambiante, le virus est dilué par du milieu et l'infection poursuivie en étuve à 37°C. Après différents temps d'infection (3 heures à 72 heures) les surnageants sont congelés et les cellules, après 2 lavages successifs, sont grattées et congelées en milieu + BSA (O, 1 mg/ml). Parallèlement, les virus (sauvage ou recombinant) sont titrés par coloration au rouge neutre des plages de lyse observées aux différentes multiplicités d'infection.

Titration de l'interféron produit

Pour que les virus de la vaccine présents dans les extraits n'interfèrent pas dans la titration, les extraits sont soit traités pendant un temps suffisant aux ultraviolets pour inactiver plus de 99 % du virus ; soit clarifiés par centrifugation pendant 2 heures à 30000 rpm et 4°C (condition suffisante pour culotter plus de 99,9 % du virus et récupérer plus de 95 % de l'interféron présent).

Les surnageants ne subissent pas d'autres manipulations. Les extraits cellulaires sont obtenus après éclatement des cellules par trois congélations/décongélations successives, suivies d'un traitement court aux ultrasons, puis clarification par centrifugation (10' à 10000 rpm et 4°C).

L'activité interféron, présente dans les surnageants ou les cellules ainsi traitées, est titrée par la mesure de son effet d'inhibition sur la réplication du VSV (virus de la stomatite vésiculaire) dans les cellules WISH (cellules épithéliales humaines).

Des monocouches confluentes de WISH sont incubées en présence de dilutions sérielles des extraits à tester et d'un interféron étalon (interféron naturel obtenu à partir de lymphocytes stimulés à la  PHA  ) pendant 24 heures à 37°C. Les milieux sont alors éliminés et les cellules infectées par du VSV à 0,1 MOI en milieu appauvri en sérum. Après 24 heures à 30 heures d'incubation à 37°C, temps nécessaire et suffisant à la lyse complète dans les cultures témoins, la dilution qui donne la demi-protection contre la lyse dans les extraits est comparée à celle observée avec l'interféron étalon du laboratoire.

Cet étalon interne est titré par rapport à l'étalon international (NIH G9 23-901-530), IFN-γ humain naturel, obtenu du "National Institute of Allergy and Infectious Diseases" (Bethesda, USA), pour lequel la demi-protection est observée à 1 U/ml. Les résultats sont exprimés en unités internationales.

Lors des immunotests, des dilutions sérielles d'anticorps anti-IFN-γ (anticorps monoclonal de souris préparé contre l'interféron-γ humain cloné dans les bactéries), sont préincubées pendant 2 heures à 37°C en présence d'une quantité constante d'extrait correspondant à 10 U/ml d'interféron, avant d'être rajoutées aux cellules.

Les tests de spécificité d'espèce sur lignée bovine (MDBK) ou murine (LM) se font également par infection au VSV, avec comme témoin, soit de l'interféron-α humain cloné dans les bactéries, soit de l'interféron-γ murin naturel partiellement purifié.

Pour tester la sensibilité à pH acide de l'interféron les extraits sont ajustés à pH = 2 avec de l'HCl 1 M, incubés 2 heures à 4°C puis neutralisés par addition de NaOH 1 M avant d'être titrés.

Résultats

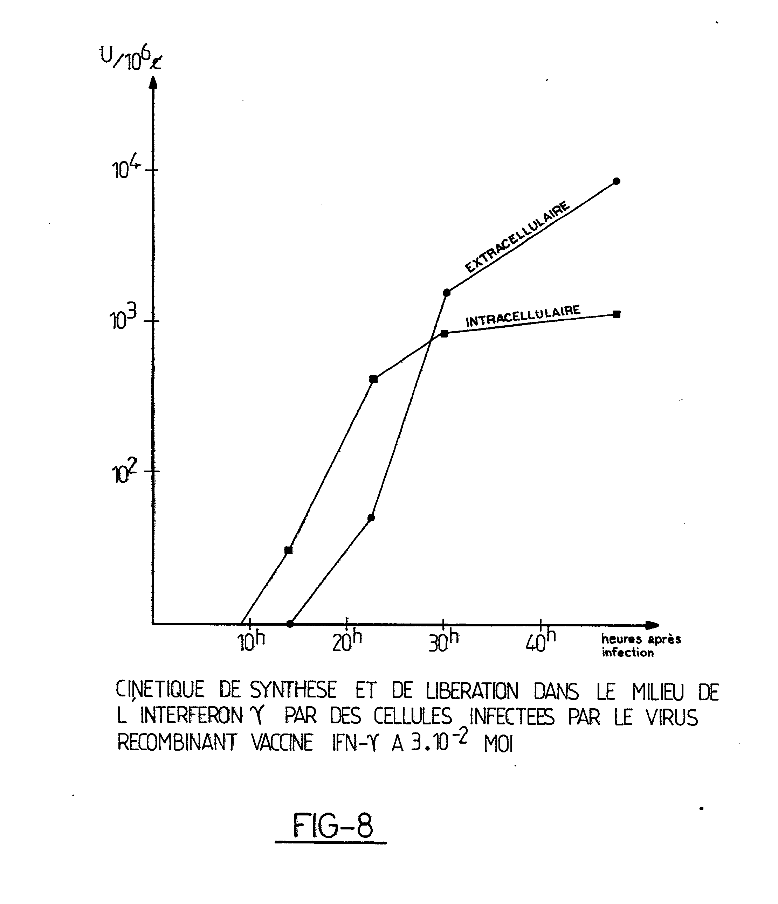

Les résultats obtenus sur la lignée Vero sont résumés dans les tableaux I, II et III et dans la figure 8.

Ils indiquent que la cinétique d'accumulation extracellulaire de l'interféron-$\gamma$ est fonction de la multiplicité d'infection du virus recombinant (tableau I) :

TABLEAU I

ETUDE CINETIQUE DE SECRETION D'INTERFERON-$\gamma$ PAR LES CELLULES VERO EN FONCTION DE LA MULTI- PLICITE D'INFECTION (MOI) DU VIRUS RECOMBINANT

| MOI | Temps après infection | | | | | |
|---|---|---|---|---|---|---|
| | 20 heures | | 30 heures | | 48 heures | |
| | $U/10^6$ cell. | %/max | $U/10^6$ cell. | %/max | $U/10^6$ cell. | %/max |
| 10 | $1,9 \ 10^4$ | 55 | $3,4 \ 10^4$ | 100 | $3,5 \ 10^4$ | 100 |
| 1 | $8,7 \ 10^3$ | 25 | $2,7 \ 10^4$ | 70 | $3,6 \ 10^4$ | 102 |
| 0,1 | $5 \ 10^3$ | 15 | $1,7 \ 10^4$ | 50 | $2,8 \ 10^4$ | 80 |
| $10^{-2}$ | $2 \ 10^3$ | 6 | ND | ND | $5 \ 10^3$ | 14 |
| $10^{-3}$ | $1,7 \ 10^2$ | 0,5 | ND | ND | $1,5 \ 10^3$ | 4,5 |
| $10^{-4}$ | $0,35 \ 10^2$ | 0,1 | ND | ND | $9 \ 10^2$ | 2,6 |
| $10^{-5}$ | $0,35 \ 10^2$ | 0,1 | ND | ND | $1,75 \ 10^2$ | 0,5 |

ND = non déterminé

A faible MOI ($< 10^{-2}$) très peu d'interféron est détecté même 48 heures après infection.

0206920

Pour des MOI $\geqslant 10^{-2}$, la quantité d'interféron extracellulaire est fonction du temps d'incubation ; la quantité maximale obtenue avec la lignée Vero (3,5 $10^4$ U/$10^6$ cell.) est obtenue dès 25 heures à 10 MOI alors qu'elle ne l'est pas avant 48 heures à 1 MOI.

En se plaçant à une MOI moyenne (3 $10^{-2}$), on peut également visualiser les processus successifs de synthèse intracellulaire de l'interféron, puis de libération dans le milieu extracellulaire (figure 8). Après 48 heures d'infection, 10 à 30 % de l'interféron produit reste intracellulaire et ce quelle que soit la MOI.

On vérifie que l'interféron synthétisé par la lignée Vero infectée par le virus vaccine recombinant présente les propriétés spécifiques d'un interféron-γ humain :

- Spécificité d'espèce vis-à-vis d'une lignée cellulaire humaine, c'est-à-dire aucune activité antivirale sur lignée bovine ou murine (tableau II).

- Sensibilité à la température, à l'acidité et aux détergents (tableau III).

- Neutralisation par un anticorps spécifiquement dirigé contre l'interféron-γ humain (tableau II).

## TABLEAU II

### CARACTERISATION DE L'INTERFERON PRODUIT DANS LES CELLULES VERO INFECTEES PAR LE VIRUS RECOMBINANT VACCINE IFN-γ

| Critère | Surnageant de cellules Vero infectées par le virus recombinant | IFNγ humain standard NIH | IFNγ humain standard interne | IFNγ humain cloné dans E. coli | IFNα humain cloné dans E. coli |
|---|---|---|---|---|---|
| Titration sur cellules humaines $(U.ml^{-1})$ | $10^4$ | $4 \ 10^3$ | $5 \ 10^2$ | $10^4$ | $7 \ 10^3$ |
| Titration sur cellules bovines $(U.ml^{-1})$ | < 80 | 120 | < 10 | < 200 | $7 \ 10^3$ |
| Neutralisation par anticorps anti-IFNγ humain (titre de l'anticorps contre 10 unités) | $1,2 \ 10^3$ | $1,5 \ 10^3$ | $1,3 \ 10^4$ | $7 \ 10^3$ | < 8 |

17

TABLEAU III

STABILITE DE L'INTERFERON PRODUIT DANS LES
CELLULES VERO INFECTEES PAR LE VIRUS RECOMBINANT VACCINE IFN

| Traitement | Surnageant de cellules vero infectées par le virus recombinant | IFN-γ humain standard interne | IFN-γ humain cloné dans E. coli | IFN-α huma cloné dan E. coli |
|---|---|---|---|---|
| contrôle | 100 | 100 | 100 | 100 |
| pH:2 2h à 4°C | < 0,3 | < 0,6 | 3 | 100 |
| SDS 0,1 % 2h à 4°C | < 0,6 | < 3 | 12 | 100 |
| 56°C, 1h | 1,2 | < 0,6 | < 0,1 | ND |

ND = non déterminé

Les résultats sont exprimés en % d'activité par rapport
au contrôle.

Conclusion

Toutes les lignées testées se sont révélées capables
de synthétiser l'interféron-γ humain après infection par un
virus de la vaccine recombinant.

On a pu vérifier que sur ces lignées, y compris
les lignées humaines, l'interféron-γ humain produit avait
un effet négligeable sur la réplication de la vaccine,
dans les conditions d'infection utilisées.

Les titres maximum obtenus sur ces différentes lignées
se situent entre $2 \cdot 10^4$ et $7 \cdot 10^4$ unités d'interféron-γ
humain pour $10^6$ cellules infectées ; ce facteur 3 semble
refléter des sensibilités légèrement différentes des lignées
au virus de la vaccine.

En prenant en compte une activité spécifique décrite pour l'interféron naturel de $10^8$ U/mg, ces résultats indiquent que l'on peut attendre une production maximale de 0,2 à 0,7 µg pour $10^6$ cellules infectées, soit environ 0,2 à 0,7 mg d'interféron purifié par litre de culture.

Le plasmide pVVTG41 a été déposé le 12 juin 1985 à la Collection Nationale de Cultures de Microorganismes de l'Institut Pasteur, 28 rue du Docteur-Roux - 75015 PARIS, sous le numéro suivant :

. E. coli souche    5K pVVTG41 : n° I-456.

La description de ce plasmide est la suivante :

Plasmide de réplication de E. coli portant un cADN de l'interféron γ humain (IFN-γ) placé sous le contrôle d'un promoteur du virus de la vaccine et destiné à la recombinaison in vivo, dans les cellules de mammifères, avec le virus de la vaccine, pour engendrer un virus recombinant vaccine TK-IFN-γ, capable d'exprimer l'IFN-γ dans des cellules de mammifères.

- le cADN de l'IFN-γ a été isolé d'une banque provenant d'ARN messager de lymphocytes stimulés par un agent mitogène.

- Ce cADN a été placé sous le contrôle d'un promoteur du virus de la vaccine, le promoteur de la protéine 7,5 k.

- La séquence d'ADN P 7,5k-IFN-γ a été intégrée dans le gène TK de la vaccine pour favoriser une recombinaison homologue avec le gène TK du virus de la vaccine de type sauvage qui sera utilisé pour infecter les cellules de mammifères.

- La séquence d'ADN comprenant P7,5K-IFN-γ intégrée dans le gène TK a été insérée dans un plasmide de E. coli dérivé du pML2 et comprenant une origine de réplication dans E. coli et le gène de la β-lactamase.

Cette construction s'appelle pVVTG41.

L'ADN du plasmide pVVTG41 sera utilisé sous forme de précipité de phosphate de calcium pour transfecter des cellules de mammifères infectées par le virus de la vaccine et obtenir ainsi, au cours de la multiplication virale dans la cellule, une recombinaison entre le gène TK du virus et le gène TK contenant le gène IFN-γ, porté par le plasmide. Le virus recombinant résultant de cet échange sera TK⁻ et portera le gène de l'IFN-γ.

0206920

## REFERENCES

1. Drillien R. & Spehner D. (1983) Virology 131, 385-393.

2. Blalock J.E., Georgiades J.A., Longford M.P. & Johnson H.M. (1980) Cell Immunol. 49, 390-394.

3. Glasgow L.A., Crane J.L., Jern E.R. & Younger J.S. (1978) Cancer Treat. Rep. 62, 1881-1888.

4. Gray P.W., Yeung D.W., Pennica D., Yelverton E., Najatian R., Simonsen C.C., Derynck R., Sherwood P.J., Wallace D.M., Berger S.L., Levinson A.D. & Goeddel D.V. (1982) Nature (London) 295, 503-508.

5. Isaacs A., Lindenmann J. (1957) Proc. Royal Soc. B147, 258-267.

6. Kieny M.P., Lathe R. & Lecocq J.P. (1983) Gene 26, 91-99.

7. Kieny M.P., Lathe R., Drillien R., Spehner A., Skory S., Schmitt D., Wiktor T., Koprowski H. & Lecocq J.P. Mat., (1984) Nature, 312, 163-166.

8. Lathe R., Kieny M.P., Skory S. & Lecocq J.P. (1984) DNA 3, 173-182.

9. Lusky M., Botchan M. (1981) Nature 293, 79-81.

10. Mackett M., Smith J.L. & Moss B. (1982) Proc. Natl. Acad. Sci. USA 79, 7415-7419.

11. Panicali D. & Paoletti E. (1982) Proc. Natl. Acad. Sci. USA 79, 4927-4931.

12. Panicali D., Davis S.W., Weinberg R.L. & Paoletti E. (1983) Proc. Natl. Acad. Sci. USA 80, 5364-5368.

13. Rinderknecht E., O'Connor B.H. & Rodriguez H. (1984) Journ. Biol. Chem. 259, 6790-6797.

14. Salvin B.S., Younger J.S., Nishio H. & Meta R. (1975) J. Natl. Cancer Inst. 55, 1233-1236.

15. Smith G.L., Mackett M. & Moss V. (1983) Nature 302, 490-495.

16. Sonnenfeld G., Mandel A.D. & Merigan T.C. (1977) Cell Immunol. 34, 193-206.

17. Southern E.M. (1975) J. Molec. Biol. 98, 503.

0206920

18. Stewart W.E. (1979) "The Interferon System", Springer Verlag, New-York.

19. Venkatesan S., Baroudy B.M. & Moss B. (1981) Cell 125, 805-813.

20. Vieira J. & Messing J. (1982) Gene 19, 259-268.

21. Weir J.P. & Moss B. (1983) J. Virol. 46, 530-537.

0206920

## REVENDICATIONS

1) Poxvirus caractérisé en ce qu'il comporte tout ou partie d'une séquence d'ADN codant pour IFN-γ.

2) Poxvirus selon la revendication 1, caractérisé en ce qu'il s'agit du virus de la vaccine.

3) Virus selon la revendication 2, caractérisé en ce que la séquence d'ADN codant pour IFN-γ est la séquence d'ADN codant pour la protéine complète.

4) Virus selon l'une des revendications 1 à 3, caractérisé en ce que la séquence d'ADN codant pour IFN-γ est sous la dépendance d'un promoteur d'un gène du poxvirus

5) Virus selon la revendication 4, caractérisé en ce que le promoteur est un promoteur du gène de la vaccine.

6) Virus selon la revendication 5, caractérisé en ce que la séquence d'ADN codant pour IFN-γ est sous le contrôle du promoteur du gène de la protéine 7,5K de la vaccine.

7) Virus selon l'une des revendications 1 à 6, caractérisé en ce que la séquence codant pour IFN-γ est clonée dans le gène TK de la vaccine.

8) Virus recombinant pVVTG41.

9) Cellule de mammifères infectée par un virus selon l'une des revendications 1 à 8.

10) Procédé de préparation d'IFN-γ , caractérisé en ce qu'on cultive des cellules selon la revendication 9 et que l'on récupère la protéine formée.

11) IFN-γ obtenu par la mise en oeuvre du procédé selon la revendication 10.

12) IFN-γ obtenu à partir de culture cellulaire, caractérisé en ce qu'il est sous forme mature et glycosylée.

24

0206920

13) Composition thérapeutique, caractérisée en ce qu'elle comporte l'IFN-γ selon la revendication 11 ou 12.

14) Composition thérapeutique, caractérisée en ce qu'elle comporte un virus selon l'une des revendications 1 à 8.

15) Composition selon la revendication 14, caractérisée en ce qu'elle est sous forme inoculable.

0206920

1 / 8    Bgl II

GA
↑↑

GGGGGGGGGGGGGAACTTCTTTGGCTTAATTCTCTCGGAAACG

ATG AAA TAT ACA AGT TAT ATC TTG GCT TTT
Met Lys Tyr Thr Ser Tyr Ile Leu Ala Phe

CAG CTC TGC ATC GTT TTG GGT TCT CTT GGC
Gln Leu Cys Ile Val Leu Gly Ser Leu Gly

TGT TAC TGC CAG GAC CCA TAT GTA AAA GAA
Cys Tyr Cys Gln Asp Pro Tyr Val Lys Glu

GCA GAA AAC CTT AAG AAA TAT TTT AAT GCA
Ala Glu Asn Leu Lys Lys Tyr Phe Asn Ala

GGT CAT TCA GAT GTA GCG GAT AAT GGA ACT
Gly His Ser Asp Val Ala Asp Asn Gly Thr

CTT TTC TTA GGC ATT TTG AAG AAT TGG AAA
Leu Phe Leu Gly Ile Leu Lys Asn Trp Lys

GAG GAG AGT GAC AGA AAA ATA ATG CAG AGC
Glu Glu Ser Asp Arg Lys Ile Met Gln Ser

CAA ATT GTC TCC TTT TAC TTC AAA CTT TTT
Gln Ile Val Ser Phe Tyr Phe Lys Leu Phe

AAA AAC TTT AAA GAT GAC CAG AGC ATC CAA
Lys Asn Phe Lys Asp Asp Gln Ser Ile Gln

AAG AGT GTG GAG ACC ATC AAG GAA GAC ATG
Lys Ser Val Glu Thr Ile Lys Glu Asp Met

AAT GTC AAG TTT TTC GAT AGC AAC AAA AAG
Asn Val Lys Phe Phe Asp Ser Asn Lys Lys

AAA CGA GAT GAC TTC GAA AAG CTG ACT AAT
Lys Arg Asp Asp Phe Glu Lys Leu Thr Asn

TAT TCG GTA ACT GAC TTG AAT GTC CAA CGC
Tyr Ser Val Thr Asp Leu Asn Val Gln Arg

AAA GCA ATA CAT GAA CTC ATC CAA GTG ATG
Lys Ala Ile His Glu Leu Ile Gln Val Met

GCT GAA CTG TCG CCA GCA GCT AAA ACA GGG
Ala Glu Leu Ser Pro Ala Ala Lys Thr Gly

AAG CGA AAA AGG AGT CAG ATG CTG TTT CGA
Lys Arg Lys Arg Ser Gln Met Leu Phe Arg

GGT CGA AGA GCA TCC CAG TAA TGG TTG TCC
Gly Arg Arg Ala Ser Gln *** Trp

553
TGC CTG CAA TAT TTG AAT TTT AAA TCT AAA

583
TCTATTTATTAATATTTAACATTATTTAT

613
ATGGGGAATATATTTTTAGACTCATCAATC

643
AAATAAGTATTTATAATAGCAACTTTTGTG

673
TAATGAAAATGAATATCTATTAATATATGTA

703
TTATTTATAATTCCTATATCCTGTGACTG

733
TCTCACTTAATCCTTTGTTTTCTGACTAAT

763
TAGGCAAGGCTATGTGATTACAAGGCTTTA

793
TCTCAGGGGCCAACTAGGCAGCCAACCTAAG

823
CAAGATCCCATGGGTTGTGTGTTTATTTC

853
ACTTGATGATACAATGAACACTTATAAGTG

883
AAGTGATACTATCCAGTTACTGCCGGTTTG

913
AAAATATGCCTGCAATCTGAGCCAGTGCTTT

943
AATGGCATGTCAGACAGAACTTGAATGTG

973
TCAGGTGACCCTGATGAAAACATAGCATC

1003
TCAGGAGATTTCATGCCTGGTGCTTCCAAA

1033
TATTGTTGACAACTGTGACTGTACCCAAATGG

1063
AAAGTAACTCATTTGTTAAAATTATCAAT

1093
ATCTAATATATATGAATAAAGTGTAAGTT

1123
CACAACTAAAAAAAAAAAAAAAAAAAACCCCC

1153
CCCCCCCCC

fMet  Lys

....(G)AACTTCTTTGGCTTAATTCTCTCGGAAACGA.ATG.AAA....
n

TGGCTTAGATCTCTCGGA

MUTATION  DE  DEUX  BASES  PAR  UN  18 – MER
QUI  EST  HYBRIDE  AVEC   LE  BRIN  SIMPLE  D'UN  DERIVE
DE  M 13mp8   CONTENANT  UN  INSERT  Pst I –Hind III  DU
CLONE  pTG 11  INCORPORANT   IFN  cDNA

Bgl II
---------
.....TTTGGCTTAGATCTCTCGGGAAA.....

FIG – 2

FIG-3

FIG-4

FIG-5

FIG-6

0206920

(1) marqueur λ Hind / Eco

(2) fragment Sau-Sau de M13TG05

(3) fragment EcoRI-PstI de PVVtg41

(4-7) recombinants vaccine TK⁻

FIG.7

0206920

CINETIQUE DE SYNTHESE ET DE LIBERATION DANS LE MILIEU DE L INTERFERON $\gamma$ PAR DES CELLULES INFECTEES PAR LE VIRUS RECOMBINANT VACCINE IFN-$\gamma$ A 3.10$^{-2}$ MOI

FIG-8

# 0206920
Numéro de la demande

Office européen
des brevets

**RAPPORT DE RECHERCHE EUROPEENNE**

EP 86 40 1302

## DOCUMENTS CONSIDERES COMME PERTINENTS

Page 2

| Catégorie | Citation du document avec indication, en cas de besoin, des parties pertinentes | Revendication concernée | CLASSEMENT DE LA DEMANDE (Int. Cl.4) |
|---|---|---|---|
| Y | PROCEEDINGS OF THE NATIONAL ACADEMY OF SCIENCES OF THE USA, vol. 80, no. 23, décembre 1983, pages 7155-7159, Washington, US; G.L. SMITH et al.: "Construction and characterization of an infectious vaccinia virus recombinant that expresses the influenza hemagglutinin gene and induces resistance to influenza virus infection in hamsters" * En entier * | 1-10, 14,15 | |
| Y | SCIENCE, vol. 224, 27 avril 1984, pages 397-399; G.L. SMITH et al.: "Plasmodium knowlesi sporozoite antigen: Expression by infectious recombinant vaccinia virus" * En entier * | 1-10, 14,15 | |
| Y | GENE, vol. 35, no. 1/2, 1985, pages 169-177, Elsevier, Amsterdam, NL; D.B. BOYLE et al.: "Multiple-cloning-site plasmids for the rapid construction of recombinant poxviruses" * En entier * | 1-10, 14,15 | DOMAINES TECHNIQUES RECHERCHES (Int. Cl.4) |
| X | EP-A-0 077 670 (GENENTECH INC.) * En entier * | 11-13 | |

-/-

Le présent rapport de recherche a été établi pour toutes les revendications

| Lieu de la recherche | Date d'achèvement de la recherche | Examinateur |
|---|---|---|
| LA HAYE | 08-09-1986 | DESCAMPS J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

Office européen
des brevets

**RAPPORT DE RECHERCHE EUROPEENNE**

0206920
Numéro de la demande

EP   86  40  1302

| DOCUMENTS CONSIDERES COMME PERTINENTS | | | Page   3 |
|---|---|---|---|
| Catégorie | Citation du document avec indication, en cas de besoin. des parties pertinentes | Revendication concernée | CLASSEMENT DE LA DEMANDE (Int. Cl.4) |
| Y |  | 1-10, 14,15 |  |
|  | --- |  |  |
| X | EP-A-0 088 540   (BIOGEN N.Y.) * En entier * | 11-13 |  |
| Y |  | 1-10, 14,15 |  |
|  | --- |  |  |
| X | PROCEEDINGS OF THE NATIONAL ACADEMY OF SCIENCES OF THE USA, vol. 81, no. 16, août 1984, pages 5086-5090, Washington, US; R. FUKUNAGA et al.: "Constitutive production of human interferons by mouse cells with bovine papillomavirus as a vector" * En entier * | 11-13 | DOMAINES TECHNIQUES RECHERCHES (Int. Cl.4) |
| Y | IDEM | 1-10, 14,15 |  |
|  | --- |  |  |
|  | ----- |  |  |

Le présent rapport de recherche a été établi pour toutes les revendications

| Lieu de la recherche | Date d'achèvement de la recherche | Examinateur |
|---|---|---|
| LA HAYE | 08-09-1986 | DESCAMPS J.A. |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,Y | NATURE, vol. 312, no. 5990, novembre 1984, pages 163-166, Reading, Berks, GB; M.P. KIENY et al.: "Expression of rabies virus glycoprotein from a recombinant vaccinia virus" * En entier * | 1-10, 14,15 | C 12 N   15/00<br>C 12 P   21/02<br>A 61 K   45/02<br>A 61 K   35/76<br>C 12 N    5/00<br>C 12 N    7/00 |
| | --- | | |
| Y | EP-A-0 110 385   (THE UNITED STATES OF AMERICA) * En entier * | 1-10, 14,15 | |
| | --- | | |
| Y | JOURNAL OF VIROLOGY, vol. 49, no. 3, mars 1984, pages 857-864, American Society for Microbiology; M. MACKETT et al.: "General method for production and selection of infectious vaccinia virus recombinants expressing foreign genes" * En entier * | 1-10, 14,15 | |
| | ---              -/- | | |

| | DOMAINES TECHNIQUES RECHERCHES (Int. Cl.4) |
|---|---|
| | C 12 N<br>C 12 P<br>A 61 K |

Le présent rapport de recherche a été établi pour toutes les revendications

| Lieu de la recherche | Date d'achèvement de la recherche | Examinateur |
|---|---|---|
| LA HAYE | 08-09-1986 | DESCAMPS J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82